# EUROPEAN PATENT APPLICATION

(11) **EP 0 701 837 A1**
(43) Date of publication of application: **20.03.1996**
(21) Application number: 95306388.0
(22) Date of filing: 12.09.1995
(51) Int. Cl.: A61M 39/10

(54) **Threaded connector for a medical device**

(30) Priority: 16.09.1994 US 308055
(71) Applicant: Becton Dickinson and Company, Franklin Lakes, New Jersey 07417-1880 (US)
(72) Inventor: Peterson, Gerald H., Salt Lake City, Utah (US); Edwards, Floyd V., Sandy, Utah (US); Byron, M. Parke, Salt Lake City, Utah (US)
(74) Representative: Ruffles, Graham Keith

(57) **Abstract**

The threaded connector of this invention meets the ISO 594-2 Luer Lock Connector standard. This threaded connector is a modified female 6% Luer lock conical fitting with external threads. The threaded connector of this invention defines a pair of external threads, the mid points of which are located 180 degrees apart. Each of these threads extends only about 140 degrees to about 170 degrees around the outer surface of the tubular body defining the connector. The ends of the threads taper smoothly into the outer surface of the connector.

## Description

### Background of the Invention

This invention relates to a threaded connector for a medical device. Most medical devices contain some type of connector on one end to allow another medical device to be temporarily connected to the first medical device. For example, catheters may have a Luer lock connector on the proximal end of the catheter hub. These connectors allow another medical device such as a syringe or fluid supply line that has a complementary mating surface to the Luer lock connector to be connected to the catheter. Indeed, the need for such a connector on a medical device resulted in a standard design for the Luer lock connector. The International Standards Organization (ISO) promulgated the ISO 594-2 Luer Lock Connector standard to ensure that manufacturers of medical devices will have compatible connectors that allow different medical devices to be temporarily coupled together. This standard requires certain dimensions and angles for the connectors to insure compatibility among several different devices. This standard includes an exception for semi-rigid materials.

Unfortunately, not all manufacturers of medical devices fully comply with the ISO dimensional standard. Some manufacturers provide connectors that fall outside of the ISO dimensional requirements but that still purportedly function as a "standard" Luer lock connector. As a result, connectability between a medical device with such a connector and another medical device with a truly standard Luer lock connector may be compromised.

Some connectors on catheters are deficient because when the catheter is taped to a patient's skin the threads on the connector can abrade the skin causing potential discomfort when connected to a non-locking female luer slip-type connector, leaving the threads exposed.

### Summary of the Invention

It is therefore an object of this invention to provide a threaded connector for a medical device that complies with the ISO 594-2 Luer Lock Connector Standard.

It is another object of this invention to provide a threaded connector for a medical device that minimizes connectability problems with connectors for other medical devices that do not fully comply with the ISO standards.

It is yet another object of this invention to provide a threaded connector that minimizes site trauma to a patient.

The threaded connector of this invention is a modification of the standard female 6% Luer lock conical fitting with external threads, where the threads have been truncated and the ends of the threads have been tapered so they do not extend completely around the entire circumference of the connector. Instead, the connector of this invention defines a pair of external threads, the mid points being located about 180 degrees apart. These threads extend only around about 140 degrees or about 170 degrees around the circumference of the connector. Furthermore, the connector creates a shoulder with the medical device to which it is attached to allow the medical device to be carried by this shoulder during processing with automated production equipment.

The above and other objects and advantages of the invention will be apparent upon consideration of the drawings and the following detailed description.

### Brief Description of the Drawings

The preferred embodiments are illustrated in the drawings in which like reference numerals refer to like elements and in which:
FIG. 1 is a perspective view of the threaded connector of this invention used on a straight catheter hub as seen from the proximal end of the catheter;
FIG. 2 is a side elevation view of the threaded connector of this invention used on a straight catheter hub;
FIG. 3 is an end view of the threaded connector of this invention used on a straight catheter hub with threads that extend about 170° around the catheter hub;
FIG. 4 is an end view of the threaded connector of this invention used on a straight catheter hub with threads that extend about 140° around the catheter hub;
FIG. 5 is a perspective view of the threaded connector of this invention used on a winged catheter hub as seen from the proximal end of the winged catheter; and
FIG. 6 is an end view of the threaded connector of this invention used on a winged catheter.

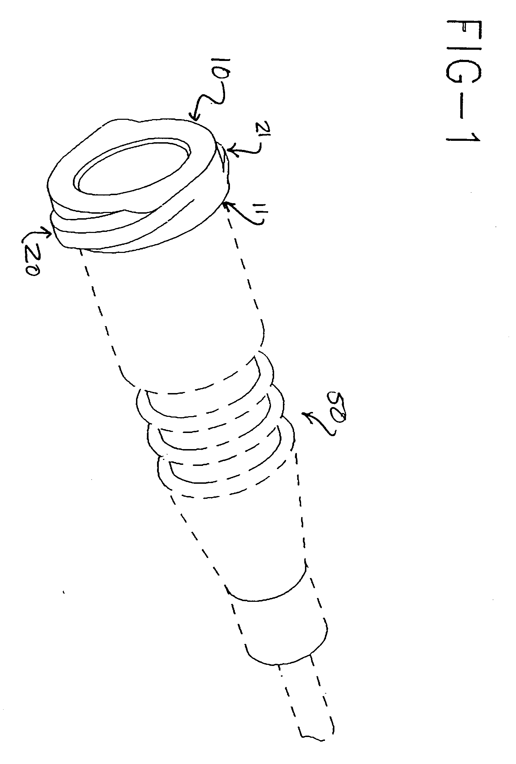

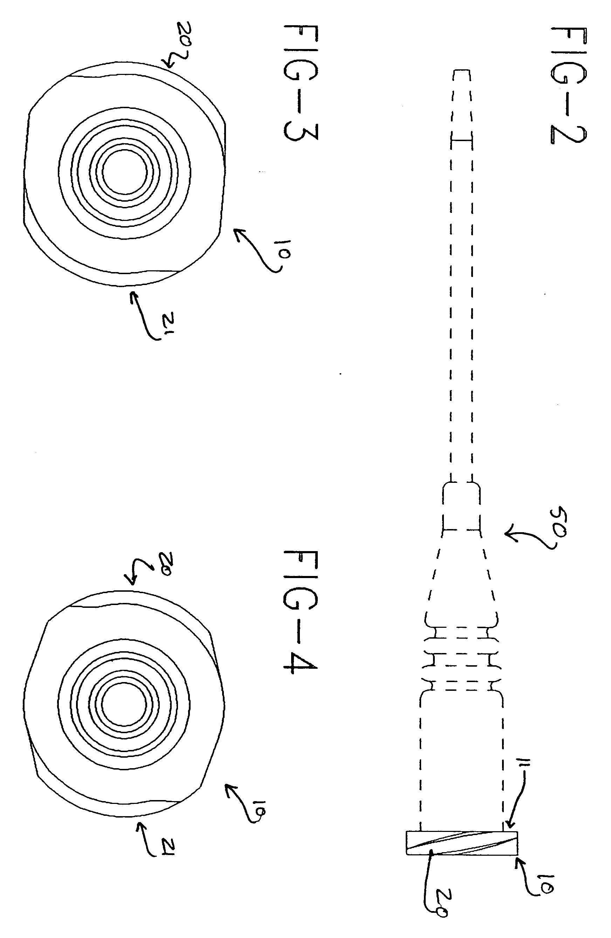

### Detailed Description of the Invention

The threaded connector of this invention is described herein and depicted in the Figures as part of an intravenous (IV) catheter. However, it is to be understood that this connector can be used in conjunction with other medical devices that require simple and secure connection to other devices.

The threaded connector 10 is located on the end of the medical device 50 to allow easy coupling of a second device thereto. Threaded connector 10 defines a shoulder 11 between threaded connector 10 and medical device 50.

Threaded connector 10 includes two threads 20 and 21 which extend partially around the circumference of threaded connector 10 on the outer surface thereof. Preferably threads 20 and 21 are on opposite sides of threaded connector, i.e. their mid points are about 180 degrees apart. Threads 20 and 21 extend between about 140 degrees to about 170 degrees around the circumference of threaded connector 10. In addition, the threads have been truncated and tapered so threaded connector 10 defines two portions on the circumference of connector 10 that are about 180 degrees apart with no threads thereon. The taper should flow smoothly into the surface of connector 10 having no threads. These portions of no threads extend around about 10 degrees to about 40 degrees on each side of the connector. The smooth taper coupled with the region of no threads allows medical device 50, which in this case is a catheter, to be taped to the patient's skin without any threads abrading or "digging in" to the patient's skin when medical device 50 is connected to another medical device having a Luer slip type of a connector.

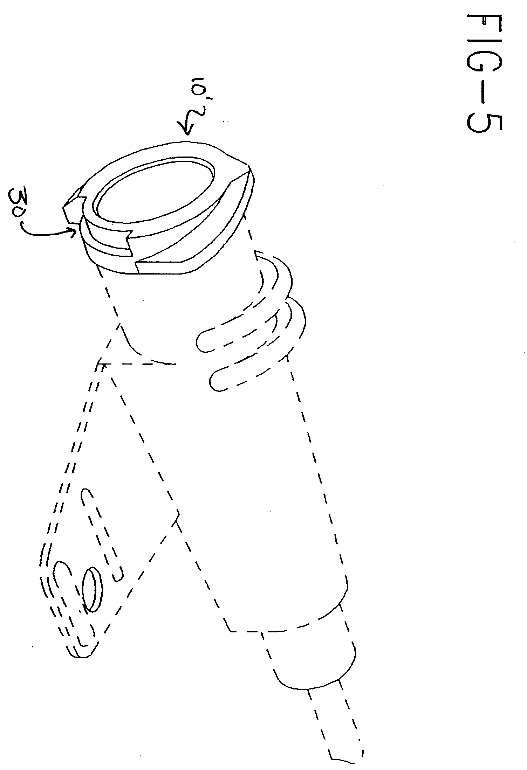

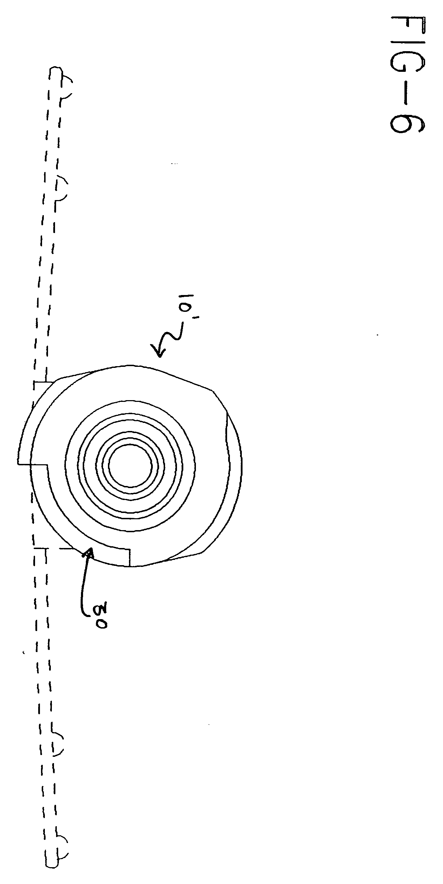

Threads 20 and 21 preferably have angled sidewalls that are not perpendicular to the outer surface of threaded connector 10. Instead, the sidewalls should be at an angle of about 26 degrees to a plane perpendicular to the outer surface of threaded connector 10. In addition FIGS. 5 and 6 show a variation of the threaded connector of this design on a catheter hub for a winged catheter. Threaded connector 10' includes a cut-out portion 30. This cut-out portion 30 mates with a tab on an introducer needle (not shown) to be used with the catheter to ensure that the bevel tip of the needle faces away from the patient's skin when the wings are placed against the patient's skin. Such a cut-out portion is used on Becton Dickinson and Company's Insyte-W® catheter.

Each of these embodiments achieves the desired goal of providing a threaded connector for a medical device that complies with the ISO 594-2 Luer Lock Connector standard, that minimizes connectability problems with connectors that do not fully comply with this ISO standard and that minimizes site trauma to the patient.

## Claims

1. A threaded connector for a medical device having a generally hollow tubular configuration and an end for receiving a second medical device therein, comprising:
a tubular body having an outer surface; and
two threads located about the outer surface of the tubular body so that the two threads are about 180 degrees apart and each thread extends between about 140 degrees to about 170 degrees around the tubular body, and wherein the two threads each have at least one end that is tapered and flows smoothly into the contour of the outer surface of the tubular body.

2. The threaded connector of Claim 1 wherein the outer surface of the tubular body defines a cut-out portion therein between the two threads.
